(19) Europäisches Patentamt
European Patent Office
Office européen des brevets

(11) EP 2 011 516 A1

(12) EUROPEAN PATENT APPLICATION
published in accordance with Art. 153(4) EPC

(43) Date of publication:
**07.01.2009 Bulletin 2009/02**

(21) Application number: **07742784.7**

(22) Date of filing: **24.04.2007**

(51) Int Cl.:
***A61K 47/34*** *(2006.01)* ***A61K 9/10*** *(2006.01)*
***A61K 47/42*** *(2006.01)* ***A61K 31/4745*** *(2006.01)*
***A61K 31/704*** *(2006.01)*

(86) International application number:
**PCT/JP2007/059349**

(87) International publication number:
**WO 2007/126110 (08.11.2007 Gazette 2007/45)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **24.04.2006 JP 2006119484**

(71) Applicant: **NanoCarrier Co., Ltd.**
**Kashiwa-shi, Chiba 277-0882 (JP)**

(72) Inventors:
- **AMANO, Yuko**
**Kashiwa-shi, Chiba 277-0882 (JP)**
- **KATO, Yasuki**
**Kashiwa-shi, Chiba 277-0882 (JP)**
- **HARADA, Mitsunori**
**Kashiwa-shi, Chiba 277-0882 (JP)**
- **SHIBATA, Naoya**
**Kashiwa-shi, Chiba 277-0882 (JP)**
- **SAITO, Hiroyuki**
**Kashiwa-shi, Chiba 277-0882 (JP)**

(74) Representative: **Cohausz & Florack**
**Patent- und Rechtsanwälte**
**Bleichstrasse 14**
**40211 Düsseldorf (DE)**

(54) **METHOD OF PRODUCING POLYMER MICELLE HAVING LOW MOLECULAR CHEMICAL ENCAPSULATED THEREIN**

(57) The present invention provides a method of encapsulating a low molecular weight drug in a polymer micelle, the method comprising the steps of: (a) dissolving or dispersing the drug having an electric charge in an aqueous medium; (b) preparing an aqueous medium containing a polymer micelle comprising a block copolymer having an overall hydrophobic region and a hydrophilic region, the overall hydrophobic region containing hydrophobic side chains and side chains having an electric charge opposite to that of the low molecular weight drug in random order; (c) mixing the aqueous medium having the low molecular weight drug dissolved or dispersed therein and the aqueous medium containing the polymer micelle; and (d) adjusting the pH of the mixed aqueous medium to a pH at which the encapsulation of the low molecular weight drug is stabilized.

EP 2 011 516 A1

## Description

Field of the Invention

The present invention relates to a method of encapsulating a drug in a polymer micelle by the remote loading method (or pH-gradient method).

Background Art

As a method of enhancing bioavailability of a poorly water-soluble or hydrophobic drug, a system is known in which a drug is encapsulated in particles such as a liposome and a polymer micelle. Among them, a method of using a block copolymer having a hydrophilic polymer segment and a hydrophobic polymer segment and encapsulating a drug in the micelle of the polymer through an interaction such as hydrophobic bonding ability between the hydrophobic polymer segment and the drug is drawing much attention, since it can be applied to a wide variety of drugs, and can provide micelles encapsulating drugs of a nanometer size (Patent number 2777530). It is known that neovasculature in tumor sites has voids of about 200 nm, from which particles of nanometer sizes leak out to accumulate in the tumor. It is believed that the drug-encapsulating polymer micelles of a large particle size have a lower tendency to accumulate in a tumor. Thus, the particle size is desired to be 200 nm or less, and more preferably 150 nm or less. In addition, from the standpoint of therapeutic effect, the amount of the drug encapsulated in the polymer micelle may preferably be as large as possible. Furthermore, drugs are often expensive, and thus considering the economy and production efficiency, it is desired that a drug be encapsulated in a polymer micelle at high yields.

As the method of producing a hydrophobic drug-encapsulating polymer micelle using a block copolymer which has a hydrophilic segment and a hydrophobic segment, a so-called drying method is advantageous in terms of a high encapsulation rate of the drug and the small size of micelle particles (Japanese Unexamined Patent Publication (Kokai) No. 2003-342168). In the drying method, a formulation comprising a polymer micelle encapsulating a drug with a regulated particle size is produced by dispersing or dissolving a block copolymer and a hydrophobic drug in a volatile organic solvent followed by removing the organic solvent, combining the residue thus obtained with water, and stirring it at a predetermined temperature for a period of time sufficient to attain a homogeneous dispersion of the residue thereby to produce the formulation.

However, in the drying method, a drug is generally required to be contacted with a micelle for a long period of time, e.g., overnight, and the resultant drug encapsulation rate and particle sizes are not always satisfactory. The drying method also involves use of common organic solvents, such as dichloromethane and chloroform, of which toxicity is causing much concern. Thus, a simple method of encapsulating a drug into a block copolymer without the use of a highly toxic organic solvent is greatly desired.

Disclosure of the Invention

It is an object of the present invention to provide a method that attains the formation of a low molecular weight drug-encapsulating polymer micelle having a small particle size at a high encapsulation rate in a short period of time and in a simple manner.

After intensive and extensive research, the present inventor has found that the above problem can be solved by introducing a drug into the micelle of a block copolymer comprising hydrophilic and hydrophobic regions by a so-called remote loading (pH gradient) method, and thereby has completed the present invention.

The present invention comprises the following aspects:

[1] A method of encapsulating a low molecular weight drug in a polymer micelle, the method comprising the steps of:

(a) dissolving or dispersing the low molecular weight drug having an electric charge in an aqueous medium;
(b) preparing an aqueous medium containing a polymer micelle comprising a block copolymer having an overall hydrophobic region and a hydrophilic region, the overall hydrophobic region containing hydrophobic side chains and side chains having an electric charge opposite to that of the low molecular weight drug in random order;
(c) mixing the aqueous medium having the low molecular weight drug dissolved or dispersed therein and the aqueous medium containing the polymer micelle; and
(d) adjusting the pH of the mixed aqueous medium to a pH at which the encapsulation of the low molecular weight drug is stabilized.

[2] The method according to [1] wherein the aqueous medium having the charged low molecular weight drug dissolved or dispersed therein has a pH which is outside the range of the pKa value ± 2 of the drug.

[3] The method according to [2] wherein the aqueous medium having the charged low molecular weight drug dissolved or dispersed therein has a pH which is outside the range of the pKa value ± 3 of the drug.
[4] The method according to any of [1] to [3] wherein the pH at which the encapsulation of the low molecular weight drug is stabilized is almost the same as the pKa of the low molecular weight drug.
[5] The method according to any of [1] to [4] which further comprises supplying energy to the mixed aqueous medium.
[6] The method according to any of [1] to [5] wherein the hydrophilic region of the block copolymer is polyethylene glycol (PEG).
[7] The method according to any of [1] to [6] wherein the overall hydrophobic region comprises an amino acid and/or a derivative thereof.
[8] The method according to [7] wherein the amino acid and/or the derivative thereof is glutamic acid or aspartic acid and/or a derivative thereof.
[9] The method according to [7] wherein the amino acid and/or the derivative thereof is lysine and/or a derivative thereof.
[10] The method according to any of [1] to [9] wherein the low molecular weight drug is selected from the group consisting of an anti-cancer agent, antimicrobial agent, antiviral agent, antibiotics, an anesthetic, and analgesic, in the form of an additive salt.

The present invention allows for the formation of a low molecular weight drug-encapsulating polymer micelle having a small particle size at a high encapsulation rate in a short period of time and in a simple manner. In addition, empty micelles for use in the present invention can be prepared in large scale. Accordingly, the present invention facilitates encapsulating a low molecular weight drug in the empty micelles prepared in large scale. Therefore, the present invention can also be used in the screening of the low molecular weight drug-encapsulating micelles.

Best Mode for Carrying out the Invention

Surprisingly, the present inventor has found that by introducing a low molecular weight drug into the micelle of a block copolymer comprising a hydrophilic region and an overall hydrophobic region by the remote loading method, a low molecular weight drug-encapsulating polymer micelle having a small particle size at a high encapsulation rate can be formed in a short period of time and in a simple manner.
The remote loading (pH gradient) method utilizes the transfer of a drug to be encapsulated in the dissociation equilibrium of the molecular/ionic type by pH, and is routinely used for encapsulating a drug into the liposome (for example, Hwang SH et al., Int. J. Pharm. 179: 85-95 (1999); Wang JP et al., Pharm. Res. 17: 782-787 (2000); Jia L et al., J. Pharm. Biomed. Anal. 28: 65-72 (2002); Eliaz RE et al., Cancer Res. 61: 2592-2601 (2001)). In order to encapsulate a drug into empty liposomes by the remote loading method, it generally takes about one day to prepare the empty liposomes, and at least one or two hours to encapsulate the drug into them. However, the present inventor has found that by applying the remote loading method to the encapsulation of a low molecular weight drug into empty micelles of a block copolymer comprising a hydrophilic region and an overall hydrophobic region, the low molecular weight drug can be mixed with the empty micelles and be instantly encapsulated into them.
The remote loading method (or pH gradient method) as used herein represents a method in which a low molecular weight drug entering into a micelle in the dissociated form is stably maintained by means of a gradient created between pH in the polymer micelle and pH in the external environment. For example, when a block copolymer of the present invention has a carboxyl group as a side chain in the overall hydrophobic region, a polymer micelle (empty micelle) formed from the block copolymer assumes a negative charge in a neutral to weakly basic aqueous medium such as PBS. On the other hand, doxorubicin hydrochloride with a pKa of 8.22, for example, assumes a positive charge and increases solubility in an acid condition such as a formate buffer or a citrate buffer. Thus, it is believed that by mixing the empty micelles dispersed in such an aqueous medium with a drug dissolved in an acid aqueous solvent, and then gradually increasing the pH of the solution to almost neutral, for example about pH 7.4, the resulting ionic bond with the carboxyl group will serve to encapsulate the low molecular weight drug in the micelle, and the hydrophobic portion will serve to maintain the low molecular weight drug more stably.
Specifically, the method of the present invention comprises the following steps:

(a) dissolving or dispersing the low molecular weight drug having an electric charge in an aqueous medium;
(b) preparing an aqueous medium containing a polymer micelle comprising a block copolymer having an overall hydrophobic region and a hydrophilic region, the overall hydrophobic region containing hydrophobic side chains and side chains having an electric charge opposite to that of the low molecular weight drug in random order;
(c) mixing the aqueous medium having the low molecular weight drug dissolved or dispersed therein and the aqueous medium containing the polymer micelle; and
(d) adjusting the pH of the mixed aqueous medium to a pH at which the encapsulation of the low molecular weight

drug is stabilized.

(a) Dissolution or dispersion of a drug having an electric charge in an aqueous medium

In accordance with the present invention, the low molecular weight drug that can be efficiently encapsulated into the polymer micelle may be any low molecular weight compound whose solubility or dispersibility may change with pH and includes, but not limited to, a low molecular weight drug in the form of an additive salt such as a hydrochloride or a sulfate. As used herein the term "low molecular weight" in the "low molecular weight drug" means a molecular weight of about 2000 or less, preferably 1500 or less. The "drug" as used herein is not limited to a pharmaceutical product, and is used interchangeably with a compound.

Examples of anti-cancer agents include irinotecan hydrochloride, epirubicin hydrochloride, erlotinib hydrochloride, oxycodone hydrochloride, gemcitabine hydrochloride, pirarubicin hydrochloride, fadrozole hydrochloride, doxorubicin hydrochloride, bleomycin hydrochloride, procarbazine hydrochloride, nogitecan hydrochloride, mitoxantrone hydrochloride, miboplatin hydrochloride, libromycin hydrochloride, levamisole hydrochloride, liarozole fumarate, osaterone acetate, chlormadinone acetate, goserelin acetate, exatecan mesilate hydrate, megestrol acetate, vindesine sulfate, vincristine sulfate, vinblastine sulfate, vinxaltine sulfate, peplomycin sulfate, methotrexate hydrochloride, leuprorelin acetate, imatinib mesylate, medroxyprogesterone acetate, estramustine phosphate sodium, fludarabine phosphate, miproxifene phosphate (multidrug resistance modulator: dofequidar fumarate), and the like.

Examples of antimicrobial agents, antibiotics, and antiviral agents include amorolfine hydrochloride, ciprofloxacin hydrochloride, cadrofloxacin hydrochloride, temafloxacin hydrochloride, butenafine hydrochloride, terbinafine hydrochloride, doxycycline hydrochloride, neticonazole hydrochloride, moxifloxacin hydrochloride, omoconazole nitrate, tosufloxacin tosilate, olamufloxacin mesilate, gemifloxacin mesylate, trovafloxacin mesylate, grepafloxacin hydrochloride, cefatamet pivoxil hydrochloride, cefotiam hexetil hydrochloride, cefozopran hydrochloride, cefotiam hydrochloride, cefcapene pivoxil hydrochloride, cefmatilen hydrochloride, cefmenoxime hydrochloride, tetracycline hydrochloride, demthylchlortetracycline hydrochloride, minocycline hydrochloride, sultamicillin tosilate, cefdaloxime pentexil tosilate, midecamycin acetate, amikacin sulfate, isepamicin sulfate, gentamycin sulfate, sisomycin sulfate, dibekacin sulfate, cefoselis sulfate, cefpirome sulfate, clindamycin hydrochloride, aciclovir, oseltamivir phosphate, saquinavir mesilate, nelfinavir mesilate, indinavir sulfate ethanolate, and the like.

Examples of anesthetics include bupivacaine hydrochloride, procaine hydrochloride, mepivacaine polyamp hydrochloride, mepivacaine hydrochloride, lignocaine hydrochloride, ropivacaine hydrochloride, and the like.

Examples of analgesics include oxycodone hydrochloride, dexmedetomidine hydrochloride, buprenorphine hydrochloride, tramadol hydrochloride, naratriptan hydrochloride, pentazocine hydrochloride, remifentanil hydrochloride, almotriptan malate, loperamide hydrochloride, lomerizine hydrochloride, flupirtine hydrochloride, proglumetacin maleate, dihydroergotamine mesilate, morphine sulfate, dihydrocodeine phosphate, and the like.

The amount of the low molecular weight drug is not specifically limited, but is generally 0.5 to 30% by weight, preferably 1 to 15% by weight, more preferably 1 to 10% by weight relative to the total weight of the block copolymer and the low molecular weight drug.

An aqueous medium for dissolving or dispersing the low molecular weight drug is not specifically limited unless it adversely affects the block copolymer and/or the low molecular weight drug, but may be one that can have the low molecular weight drug carrying a positive or negative charge, which is required for enhancing the solubility thereof.

Preferably, the above aqueous medium has a pH which is outside the range of the pKa value $\pm$ 2, more preferably the pKa value $\pm$ 3, of the low molecular weight drug. As used herein the term "dispersion" in "dissolution or dispersion" means a state in which a solute is homogeneously dispersed in an aqueous medium without any precipitate being formed. According to the present invention, when the solute is polymer micelles or liposomes, such a homogeneously dispersed state may also be called a solution.

(b) Preparation of an aqueous medium containing a polymer micelle comprising a block copolymer:

A polymer that can be used in forming the drug-encapsulating polymer micelle of the present invention is a block copolymer comprising a hydrophilic region and an overall hydrophobic region. These block copolymers may comprise any hydrophilic region and any hydrophobic region as long as it serves the purpose of the present invention.

"The overall hydrophobic region containing hydrophobic side chains and side chains having an electric charge opposite to that of the low molecular weight in random order" means a region which has, in addition to positively or negatively charged side chains, hydrophobic side chains in random order, such that the whole region exhibits hydrophobicity required for forming the polymer micelle core comprising a block copolymer. Although not specifically limited as long as the block copolymer can form a micelle, the ratio of the hydrophobic side chains to the side chains

having the opposite charge to that of the low molecular weight drug in the region should be preferably about 3:7 to 3:1, and in view of the amount of the low molecular weight drug encapsulated and the stability of the micelle per se, about 3:2 is more preferred.

Specific examples of block copolymers useful in the present invention include the following.

The hydrophilic region includes, but not limited to, a region derived from poly(ethylene glycol) [or poly(ethylene oxide)], polysaccharide, poly(vinyl pyrrolidone), poly(vinyl alcohol), poly(acrylamide), poly(acrylic acid), poly(methacrylamide), poly(methacrylic acid), poly(methacrylic acid), poly(methacrylic acid ester), poly(acrylic acid ester), polyamino acid, or a derivative thereof. The polysaccharide as used herein includes starch, dextran, fructan, galactan and the like. Among them, poly(ethylene glycol) segment is preferred since those having various functional groups are provided on one end thereof, and those with the region of regulated size are readily available.

On the other hand, the hydrophobic region includes, but not limited to, a poly(amino acid derivative) such as poly(aspartic acid) and/or a derivative thereof, poly(glutamic acid) and/or a derivative thereof, for example poly(β-alkylaspartate-co-aspartic acid), poly(β-allylaspartate-co-aspartic acid), poly(β-aralkylaspartate-co-aspartic acid), poly(γ-alkylglutamate-co-glutamic acid), poly(γ-aralkylglutamate-co-glutamic acid), poly(β-alkylaspartamide-co-aspartic acid), poly(β-arallylaspartamide-co-aspartic acid), poly(γ-aralkylglutamide-co-glutamic acid), as well as poly(lysine) and/or a derivative thereof.

The block copolymer for use in the present invention may comprise any combination of a hydrophilic region and an overall hydrophobic region having their respective molecular weights as long as it can form a polymer micelle in an aqueous medium (for example, an aqueous solution containing water or a buffered water or a water-miscible solvent, methanol, polyethylene glycol, a saccharide etc.). Preferred is a combination of a hydrophilic region comprising poly (ethylene glycol) and an overall hydrophobic region comprising a poly(amino acid) and/or a derivative thereof as mentioned above.

Optionally such a poly(amino acid derivative) region can be prepared from polyethylene glycol-co-polyaspartic acid benzyl ester or polyethylene glycol-co-polyglutamic acid benzyl ester, which is known per se. The polyethylene glycol-co-polyaspartic acid benzyl ester or polyethylene glycol-co-polyglutamic acid benzyl ester can be prepared by using, as an initiator, a polyethylene glycol of which one end is protected and the other end is an amino group, for example MeO-PEG-$CH_2CH_2CH_2$-$NH_2$, in an dehydrated organic solvent, and carrying out reaction by adding N-carboxy-β-benzyl-L-aspartate (BLA-NCA) or N-carboxy-γ-benzyl-L-glutamate (BLG-NCA) in such an amount that a desired degree of polymerization (the number of amino acid units) is obtained.

The block copolymer obtained as above is acetylated at its one end with acetyl chloride or acetic anhydride, and subjected to alkali hydrolysis to remove the benzyl group to prepare polyethylene glycol-co-polyaspartic acid or polyethylene glycol-co-polyglutamic acid. Then, in an organic solvent, benzyl alcohol is added in such an amount that a desired esterification rate is achieved. Subsequent reaction in the presence of a condensation agent, such as N-N'-dicyclohexylcarbodiimide (DCC) or N-N'-diisopropylcarbodiimide (DIPCI), can produce a block copolymer having partial benzylester.

The polyethylene glycol-co-polyaspartic acid benzyl ester is known to undergo β transition by alkali hydrolysis. It would be appreciated that the present invention may involve a block copolymer comprising an aspartic acid derivative that underwent β transition.

Aspartic acid and glutamic acid may be in any of optically active forms or a mixture thereof. The hydrophilic region and the overall hydrophobic region may be coupled via an ester bond, an amide bond, an imino bond, a carbon-carbon bond, an ether bond, etc.

Specifically, as block copolymers that is easy to manufacture and that can be conveniently used, there can be mentioned ones represented by the following formulae (I) and (II):

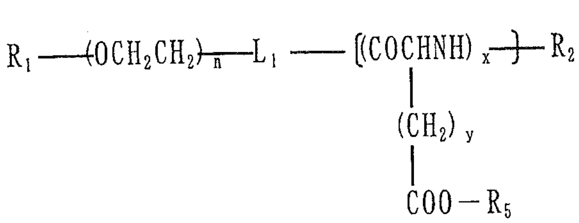

( I )

or

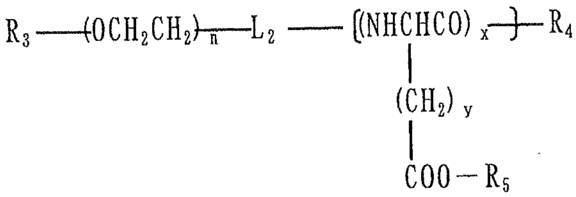

(II)

In the above formula, $R_1$ and $R_3$ each independently represent a hydrogen atom or a lower alkyl group substituted or unsubstituted with an optionally protected functional group, $R_2$ represents a hydrogen atom, a saturated or unsaturated $C_1$-$C_{29}$ aliphatic carbonyl group or an arylcarbonyl group, $R_4$ represents a hydroxy group, a saturated or unsaturated $C_1$-$C_{30}$ aliphatic oxy group or an aryl-lower alkyloxy group, and $R_5$ represents a hydrogen atom, a phenyl group, a $C_1$-$C_{16}$ alkyl group, or a benzyl group; provided that $R_5$ may be randomly selected in each amino acid unit within one block copolymer, whereas the hydrogen atom accounts for 25% to 70% relative to the total units of amino acids and is randomly present in the hydrophobic region. $L_1$ and $L_2$ each independently represent a linking group, n is an integer of 10 to 2500, x is an integer of 10 to 300, and y is an integer of 1 or 2. The optionally protected functional group includes, for example, a hydroxyl group, an acetal group, a ketal group, an aldehyde group, and a saccharide residue. The hydrophilic segment in which $R_1$ and $R_2$ represent a lower alkyl substituted with an optionally protected functional group may follow the method described in, for example, WO 96/33233, WO 96/32434, and WO 97/06202. The lower alkyl group means a linear- or branched-chain alkyl group having 7 carbons or less, preferably 4 carbons or less, and includes a methyl group, an ethyl group, a propyl group, an isopropyl group, a butyl group, an isobutyl group and the like.

The linking group is not specifically limited since it may vary with the method for producing a block copolymer, and specific examples include, for example, a group in which $L_1$ is selected from the group consisting of -NH-, -O-, -O-Z-NH-, -CO-, $-CH_2$, and -OCO-Z-NH- (Z is independently a $C_1$-$C_6$ alkylene group), and $L_2$ is selected from the group consisting of -OCO-Z-CO- and -NHCO-Z-CO-(Z is a $C_1$-$C_6$ alkylene group).

Also, the block copolymer of the present invention may have the following structure.

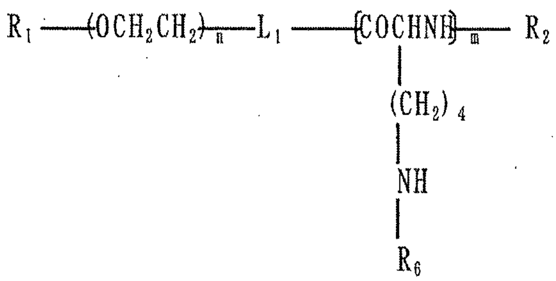

(III)

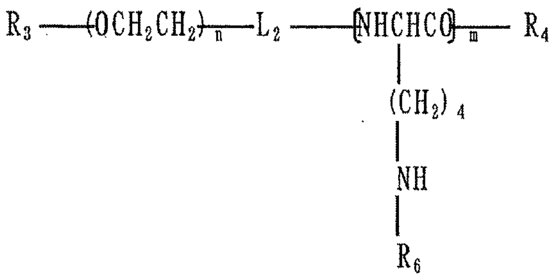

(IV)

In the above formula, $R_1$, $R_2$, $R_3$, $R_4$, and n are as defined in the above formula (I) or (II), m is an integer of 10 to 2500, $R_6$ is a phenyl group, a benzyl group, a benzoyl group, a $C_1$-$C_{16}$ alkyl group, a $C_1$-$C_{16}$ alkyl carboxylic acid, or a hydrogen atom, provided that 25% to 75% of m $R_6$ are hydrogen atoms, which are randomly present in the hydrophobic region.

An aqueous medium for dissolving or dispersing a low molecular weight drug therein is not specifically limited, but is required to have a pH such that the above drug assumes an electric charge opposite to that of the amino group (i.e., $R_6$ is a hydrogen atom) in the hydrophobic region of the above block copolymer.

Micelles may be formed, for example, by dissolving a block copolymer in a solution with stirring. Preferably,

empty micelles may be formed by applying energy such as ultrasound. The formation of empty micelles by means of ultrasound can be achieved, for example, by using Bioruptor (Nippon Seiki Co., Ltd.), and effecting irradiation at level 4, under water cooling, at intermittent intervals of one second for 5 to 6 minutes. (c) Mixing of the aqueous medium having a low molecular weight drug dissolved or dispersed therein and the aqueous medium containing the polymer micelle

The aqueous medium containing the low molecular weight drug prepared as described above is mixed with the polymer micelle-containing medium. During the mixing, it is preferred to apply energy that can promote the encapsulation of the low molecular weight drug into the polymer micelle, such as ultrasound energy. When ultrasound is used, the same condition as for the above empty micelle may be followed.

(d) Adjustment to a pH in which the encapsulation of the above low molecular weight drug in the mixed aqueous medium is stabilized

Subsequently, the pH of the resultant mixture solution is adjusted so that the low molecular weight drug may stably be retained in the micelle. The adjustment of pH may be carried out in order to minimize the electric charge of the above low molecular weight drug.

Preferably, the pH may be adjusted at a value almost equal to the pKa value of the drug, for example within ± 1, preferably within ± 0.5, and more preferably within ± 0.1 of the pKa value. Adjusting the pH of the solution at such a value reduces the solubility of the above low molecular weight drug, while the hydrophobic portion of the micelles stably retains the low molecular weight drug in the micelles.

If necessary, the aqueous solution containing the drug-encapsulating polymer micelles as obtained above may be filtered through a hydrophilic filter with a pore size of 0.22 μm. Such a 0.22 μm filter is known to be usually used in the preparation of injections (for intravenous, arterial, intramuscular, and intraperitoneal injections, etc.). Even when the above solution of the drug-encapsulating polymer micelles is aseptically filtered with a 0.22 μm filter, a sterilized aqueous solution of the drug-encapsulating polymer micelle can be obtained in an extremely high yield. Thus, in accordance with the present invention, an injection can be efficiently provided. In a preferred embodiment of the present invention, such an injection can be produced by a method further comprising the step of adding various saccharides and/or various polyethylene glycols (macrogol) to the aqueous solution of the drug-encapsulating polymer micelles before filter sterilization. Saccharides that can be used include, but not limited to, maltose, trehalose, xylitol, glucose, sucrose, fructose, lactose, mannitol, dextrin and the like, and the polyethylene glycol that can be used include those having a molecular weight of about 1,000 to about 35,000, such as macrogol 1000, 1540, 4000, 6000, 20000 and 35000. These adjuvants may be contained in the water before the above residues are combined with water, or may be added after the drug-encapsulating polymer micelles derived from the residues are dispersed or dissolved in water, after which the entire product may be aseptically filtered. Thus, in accordance with the present invention, an adjuvant that can stabilize the drug-encapsulating polymer micelles in the injection can be easily and safely added to the injection. If the pH adjusted so as to stabilize the above low molecular weight compound is not appropriate for an injection, the pH may be adjusted as appropriate immediately before administration.

In addition to the advantage of simple and same preparation, such injections have the advantage that even when they are lyophilized as a dry formulation and then redissolved or reconstituted with water into a solution containing the drug-encapsulating polymer micelle, injections that are substantially free of aggregation between micelle particles may be provided .

In order for the lyophilized formulation to exhibit the advantageous effect as described above, a saccharide in a solution before lyophilization may be added in a final concentration of 0.1 to 15%(w/v) and polyethylene glycol may be added in a final concentration of 0.5 to 10%(w/v). Generally, the ratio of a block copolymer to a saccharide or polyethylene glycol is 1:1 to 1:10 or 1:0.5 to 1:10 in terms of weight.

The present invention will now be explained in more details with reference to Comparative Examples and Examples below.

In the following description, polyethylene glycol-poly(β-benzyl-L-aspartic acid) block copolymer is abbreviated as PEG-PBLA, polyethylene glycol-poly(octylester-L-aspartic acid) block copolymer as PEG-POLA, and polyethylene glycol-poly(γ-benzyl-L-glutamic acid) block copolymer as PEG-PBLG. In addition, assuming that a block copolymer has a PEG chain whose average molecular weight is 12,000, and a poly(amino acid) chain consisting of 40 residues, and that the introduction rate of the benzyl group to the side chains of the poly(amino acid) is 60%, then each block copolymer is followed by a description 12-40(60). Similarly, when the introduction rate of octylester is 50%, it is also described 12-40(60). In the Examples below, when the introduction rate of the hydrophobic group is 60% to 65%, it is described as 60%.

Examples

Example 1: Preparation of a doxorubicin hydrochloride-encapsulating micelle formulation by the remote loading method

20 mg each of PEG-PBLA 12-50(60), PEG-PBLA 12-40(60), PEG-POLA 12-40(60), and PEG-PBLG 12-40 (60) was weighed into a screw-capped tube, combined with 3 mL of PBS (PBS Tablets, TAKARA BIO INC. were prepared according to the preparation method, the same hereinbelow), and was subjected to an ultrasound treatment to prepare polymer micelles (empty micelles). 3.5 mg of doxorubicin hydrochloride (hereinafter referred to as Dox·HCl) was dissolved in 1 mL of a formate buffer (pH 3.0) to prepare a solution, of which 200 μL was mixed with 2 mL of the empty micelle solution followed by an ultrasound treatment. Then pH was adjusted to 7.4 with 0.1 mol/L NaOH to prepare Dox·HCl-encapsulating micelles.

The particle size of the micelles prepared was measured by DLS-7000 (Ohtsuka Densi K.K.), and indicated as the average of Histogram results G (wt). The encapsulation rate was determined by carrying out gel filtration with Sephadex G-25 column (PD-10, Healthcare Bioscience) using PBS as the mobile phase, and measuring absorbance at 480 nm using the Microplate reader (Dainippon Pharmaceutical Co., Ltd.) to determine the Dox·HCl concentration of each fraction, which was inserted to the following equation:

Rate of encapsulation (%) = (Total of the amount of drug in the micelle peak) * 100 / (Total of the amount of drug in the micelle peak + Total of the amount of free drug)

The result is shown in Table 1.

Table 1

| | Particle size (nm) | Encapsulation rate (%) |
|---|---|---|
| PEG-PBLA 12-50(60) micelle | 47.3 | 64.5 |
| PEG-PBLA 12-40(60) micelle | <30.0 | 94.9 |
| PEG-POLA 12-40(60) micelle | 41.4 | 87.5 |
| PEG-PBLG 12-40(60) micelle | 45.5 | 36.2 |

The particle size was small, ranging from less than 30 nm to 47 nm, while the encapsulation rate was as high as the range of from 65% to 95%, except for PEG-PBLG 12-40(60). For PEG-PBLA 12-40(60), the micelles with a particle size of less than 30.0 nm and the encapsulation rate of 87.6% were prepared without an ultrasound treatment.

These results are compared to the results described in WO 99/61512, in which Dox·HCl was encapsulated in a block copolymer PLP (palmitoyl poly-L-lysine polyethylene glycol) and POP (palmitoyl poly-L-ornithine polyethylene glycol) without using a pH gradient. For comparison with the results of the patent publication, the encapsulation rate was determined by ultracentrifugation, the results of which are shown in Table 2. After a 150,000gx, 1 hr treatment as described in the above publication, the encapsulation rates were calculated in a similar manner to that in the present method, and was found to be 3.5% for POP, cholesterol + Dox·HCl [(in the literature, 0.014 ± 0.0042 (Dox·HCl, POP $gg^{-1}$))], and 2.9% for PLP, cholesterol + Dox·HCl [(in the literature, 0.0117 ± 0.0015 (Dox·HCl, POP $gg^{-1}$))]. The particle sizes were 361 ± 13 nm for the former, and 531 ± 56 nm for the latter. Compared with these results, the micelles prepared by the remote loading method had smaller particle sizes and much higher encapsulation rates.

Table 2

| Encapsulation rate (%) | |
|---|---|
| | PEG-PBLA 12-50(60) micelles |
| Gel filtration | 64.5 |

(continued)

| Encapsulation rate (%) | | |
|---|---|---|
| | | PEG-PBLA 12-50(60) micelles |
| Ultracentrifugation | 100,000gx 1hr | 18.4 |
| | 200,000gx 1hr | 53.5 |
| | 500,000gx 1hr | 78.4 |

Example 2: Study on the weight ratio of the drug Dox·HCl to PEG-PBLA 12-50(60)

20 mg of 12-50(60) was weighed into a screw-capped tube, combined with 3 mL of PBS (pH 7.4), and was subjected to an ultrasound treatment to prepare polymer micelles (empty micelles). Dox·HCl was dissolved in a formate buffer (pH 3.0) at a concentration of 3.5 mg/ml to prepare a solution, of which 90 μL, 130 μl, 200 μl, 400 μl, or 800 μl was taken in a screw-capped tube, and mixed with 2 mL of the empty micelle solution followed by an ultrasound treatment. Then pH was adjusted to 7.4 with 0.1 mol/L NaOH to prepare Dox·HCl-encapsulating micelles. The particle size and the encapsulation rate of the micelles prepared were measured in a similar manner as in Example 1. The results are shown in Table 3.

Table 3

| Dox·HCl:PEG-PBLA 12-50(60) (weight ratio) | 1:5 | 1:10 | 1:19 | 1:29 | 1:42 |
|---|---|---|---|---|---|
| Encapsulation rate (%) | 3.24 | 36.9 | 64.5 | 51.7 | 61.5 |
| Dox·HCl (mol) encapsulated in 1 mol of PEG-PBLA 12-50(60) | 0.2 | 1.2 | 1.1 | 0.6 | 0.5 |

Comparative Example 1: Preparation of Dox·HCl-encapsulating micelle formulation by the drying method

20 mg each of PEG-PBLA 12-50(60), PEG-PBLA 12-40(60), PEG-POLA 12-40(60), and PEG-PBLG 12-40 (60) was weighed into a screw-capped tube, combined with 1 mg of Dox·HCl, and then dissolved in 1 ml of a dichloromethane/methanol mixed solvent (1:1). After the solvent was evaporated under a nitrogen gas, 3 ml of distilled water was added, and stirred overnight at 4°C by a stirrer. After stirring, it was subjected to an ultrasound treatment to prepare Dox·HCl-encapsulating micelles. The particle size and the encapsulation rate of the micelles prepared were measured in a similar manner as in Working Example 1. The results are shown in Table 4.

Table 4

| | Particle size (nm) | Encapsulation rate (%) |
|---|---|---|
| PEG-PBLA 12-50(60) micelle | 62.7 | 23.4 |
| PEG-PBLA 12-40(60) micelle | 42.8 | 91.1 |
| PEG-POLA 12-40(60) micelle | 50.6 | 75.0 |
| PEG-PBLG 12-40(60) micelle | 57.0 | 15.5 |

Compared to the micelles (Table 1) prepared by the remote loading method, the particle sizes of the micelles prepared by the drying method are 1.2 to 1.4 fold greater. With regard to the encapsulation rates, the values of the micelles prepared by the remote loading method were equal to, or 1.2 to 2.8 fold higher than, the values of the micelles prepared by the drying method.

Comparative Example 2: Preparation of Dox·HCl-encapsulating liposome formulation (preparation in accordance with Nonpatent documents 2 and 4)

100 μl of 100 mg/mL solution of H-purified soybean lecithin (hereinafter referred to as HSPC) in dichloromethane, 24 μl of 100 mg/mL solution of cholesterol (hereinafter referred to as Chol) in dichloromethane, and 72 μl of 50 mg/mL solution of 1,2-distearoyl-sn-glycerophosphatidyl ethanolamine-N-PEG 5000 (hereinafter referred to as PEG-DSPE) in a dichloromethane/ethanol mixed solvent (1:1) were taken in a screw-capped tube (HSPC:Chol:PEG-DSPE=2:1:0.1(mol)), dried under a nitrogen gas, and further dried in a dessicator for 1.5 hour. Subsequently, 1 mL of 250 mM ammonium sulfate was added and suspended with stirring under heating at 60°C, followed by an ultrasound treatment. The resultant suspension was extruded for 11 times using the Mini-Exruder (0.1 μm PVP membrane, Avanti Polar Lipids Inc.), placed in a dialysis membrane (Spectra/por (trademark), CE, MWCO 3500), and dialyzed three times using 100 ml of 5% glucose solution as the outer liquid to prepare an empty liposome solution in an amount of about 1 mL. 800 μl of the thus-prepared empty liposome solution was mixed with 490 μl of 2 mg/mL solution of Dox·HCl in 5% glucose, and the resultant mixture was allowed to stand in a water bath at 65.0°C for 2 hours to prepare a Dox·HCl-encapsulating liposome formulation.

After the preparation, the particle size and the encapsulation rate were measured in a manner similar to that used for obtaining the results in Table 1 of Example 1. The phospholipid concentration in the liposome was determined using the C-test Kit (Wako) for phospholipid determination. As a result, the particle size was 74.3 mm, the encapsulation rate was 96.2%, and the amount of Dox·HCl (μg) per μmol of the phospholipid was 83.6 μg/μmol.

Compared to the particle size of the liposome, the particle size of the micelles prepared by the remote loading method was 1.6 to 2.5 fold smaller, while the encapsulation rate of the liposome was about the same as that of PEG-PBLA 12-40(60).

Example 3: Preparation of irinotecan hydrochloride-encapsulating micelle formulation PEG-PBLA 12-50(60) or PEG-PBLA 12-40(60)

20 mg each was weighed into a screw-capped tube, combined with 3 mL of PBS (pH 7.4), and subjected to an ultrasound treatment to prepare polymer micelles (empty micelles). 3.5 mg of irinotecan hydrochloride (hereinafter referred to as CPT-11) was dissolved in 1 mL of 50 mM citrate buffer (pH 3.5), and 200 μL of the resultant solution was mixed with 2 mL of the empty micelle solution, followed by an ultrasound treatment. Then pH was adjusted to 7.4 with 0.1 mol/L NaOH to prepare CPT-11-encapsulating micelles. In the drying method, after 20 mg each of PEG-PBLA 12-50 (60) or PEG-PBLA 12-40(60) was weighed into a screw-capped tube, combined with 1 mg of CPT-11, and then dissolved in 1 ml of dichloromethane, after which the solvent was evaporated under nitrogen gas. 3 ml of distilled water was added, and the mixture was stirred overnight at 4°C by a stirrer, and then subjected to an ultrasound treatment to prepare CPT-11-encapsulating micelles. The measurement was carried out in a similar manner as in Example 1 except that the encapsulation rate of the micelles prepared was measured by determining the absorbance at 370 nm. The results are shown in Table 5.

Table 5

| Encapsulation rate | | |
|---|---|---|
| | Remote loading method | Drying method |
| PEG-PBLA 12-50(60) micelle | 31.1 | 12.3 |
| PEG-PBLA 12-40(60) micelle | 45.5 | 23.6 |

Comparison of the encapsulation rate of the remote loading method with that of the drying method shows that the encapsulation rate of the micelles prepared by the remote loading method was 1.9 to 2.5 fold higher.

Example 4: Preparation of vincristine hydrochloride-encapsulating micelle PEG-PBLA 12-50(60) or PEG-PBLA 12-40(60)

20 mg each was weighed into a screw-capped tube, combined with 3 mL of PBS (pH 7.4), and then subjected to an ultrasound treatment to prepare polymer micelles (empty micelles). 3.5 mg of vincristine hydrochloride (hereinafter referred to as VCR) was dissolved in 1 mL of 2.5 mM formate buffer (pH 3.0) to prepare a solution, of which 200 μL was mixed with 2 mL of the empty micelle solution, followed by an ultrasound treatment. Then pH was adjusted to 7.4 with 0.1 mol/L NaOH to prepare VCR-encapsulating micelles. In the drying method, 20 mg each of PEG-PBLA 12-50(60) or PEG-PBLA 12-40(60) was weighed into a screw-capped tube, combined with 1 mg of VCR, and then dissolved in 1 ml of dichloromethane. Subsequently, the solvent was evaporated under nitrogen gas. 3 ml of distilled water was then added, and the mixture was stirred overnight at 4°C by a stirrer, and then subjected to an ultrasound treatment to prepare VCR-encapsulating micelles. The measurement was carried out in a similar manner as in Example 1 except that the encapsulation rate of the micelle prepared was measured by determining the absorbance at 300 nm. The result is shown in Table 6.

Table 6

| Encapsulation rate | | |
|---|---|---|
| | Remote loading method | Drying method |
| PEG-PBLA 12-50(60) micelle | 49.8 | 13.8 |
| PEG-PBLA 12-40(60) micelle | 73.2 | 53.7 |

The VCR encapsulation rate of the micelles prepared by the remote loading method was 1.4 to 3.6 fold higher compared to those prepared by the drying method.

## Claims

**1.** A method of encapsulating a low molecular weight drug in a polymer micelle, the method comprising the steps of:

(a) dissolving or dispersing the low molecular weight drug having an electric charge in an aqueous medium;
(b) preparing an aqueous medium containing a polymer micelle comprising a block copolymer having an overall hydrophobic region and a hydrophilic region, the overall hydrophobic region containing hydrophobic side chains and side chains having an electric charge opposite to that of the low molecular weight drug in random order;
(c) mixing the aqueous medium having the low molecular weight drug dissolved or dispersed therein and the aqueous medium containing the polymer micelle; and
(d) adjusting the pH of the mixed aqueous medium to a pH at which the encapsulation of the low molecular weight drug is stabilized.

**2.** The method according to claim 1 wherein the aqueous medium having the charged low molecular weight drug dissolved or dispersed therein has a pH which is outside the range that exceeds the pKa value ± 2 of the drug.

**3.** The method according to claim 2 wherein the aqueous medium having the charged low molecular weight drug dissolved or dispersed therein has a pH which is outside the range that exceeds the pKa value ± 3 of the drug.

**4.** The method according to any of claims 1 to 3 wherein the pH at which the encapsulation of the low molecular weight drug is stabilized is almost the same as the pKa of the low molecular weight drug.

**5.** The method according to any of claims 1 to 4 which further comprises supplying energy to the mixed aqueous medium.

**6.** The method according to any of claims 1 to 5 wherein the hydrophilic region of the block copolymer is polyethylene glycol (PEG).

**7.** The method according to any of claims 1 to 6 wherein the overall hydrophobic region comprises an amino acid and/or a derivative thereof.

**8.** The method according to claim 7 wherein the amino acid and/or the derivative thereof is glutamic acid, or aspartic acid and/or a derivative thereof.

**9.** The method according to claim 7 wherein the amino acid and/or the derivative thereof is lysine and/or a derivative thereof.

**10.** The method according to any of claims 1 to 9 wherein the low molecular weight drug is selected from the group consisting of an anti-cancer agent, an antimicrobial agent, an antiviral agent, an antibiotics, an anesthetic, and an analgesic, in the form of an additive salt.

**INTERNATIONAL SEARCH REPORT**

International application No.
PCT/JP2007/059349

A. CLASSIFICATION OF SUBJECT MATTER
*A61K47/34*(2006.01)i, *A61K9/10*(2006.01)i, *A61K47/42*(2006.01)i, *A61K31/4745*(2006.01)n, *A61K31/704*(2006.01)n

According to International Patent Classification (IPC) or to both national classification and IPC

B. FIELDS SEARCHED

Minimum documentation searched (classification system followed by classification symbols)
A61K47/34, A61K9/10, A61K47/42, A61K31/4745, A61K31/704

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN), WPIDS(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X<br>Y | CHOUCAIR, A. ET AL. 'ACTIVE LOADING AND TUNABLE RELEASE OF DOXORUBICIN FROM BLOCK COPOLYMER VESICLES.' LANGMUIR,(2005) 21(20) P.9308-9313 full text; ABSTRACT,P.9310 right column-P.9313 right column 3.RESULTS AND DISCUSSION-4.SUMMARY AND CONCLUSIONS | 1-5,10<br>1-10 |
| Y | KATAOKA, K. ET AL. 'DOXORUBICIN-LOADED POLY (ETHYLENE GLYCOL)-POLY( β -BENZYL-L-ASPARTATE) COPOLYMER MICELLES:THEIR PHARMACEUTICAL CHARACTERISTICS AND BIOLOGICAL SIGNIFICANCE.' JOURNAL OF CONTROLLED RELEASE, (2000) 64(1-3) P.143-153 full text; ABSTRACT,FIG.7 | 1-10 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>19 June, 2007 (19.06.07) | Date of mailing of the international search report<br>03 July, 2007 (03.07.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No. PCT/JP2007/059349

C (Continuation). DOCUMENTS CONSIDERED TO BE RELEVANT

| Category* | Citation of document, with indication, where appropriate, of the relevant passages | Relevant to claim No. |
|---|---|---|
| Y | GOVENDER, T. ET AL. 'DRUG-POLYIONIC BLOCK COPOLYMER INTERACTIONS FOR MICELLE FORMATION: PHYSICOCHEMICAL CHARACTERIZATION.' JOURNAL OF CONTROLLED RELEASE, (2001) 75(3) P.249-258 full text; ABSTRACT,FIG.3,TABLE2 | 1-10 |
| A | GOVENDER, T. ET AL. 'DEFINING THE DRUG INCORPORATION PROPERTIES OF PLA-PEG NANOPARTICLES.' INTERNATIONAL JOURNAL OF PHARMACEUTICS, (2000) 199(1) P.95-110 full text; P.103 right column, middle part-P.109 right column, line 14 | 1-10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2777530 A
- JP 2003342168 A
- WO 9633233 A
- WO 9632434 A
- WO 9706202 A
- WO 9961512 A


### Non-patent literature cited in the description


- **HWANG SH et al.** *Int. J. Pharm.,* 1999, vol. 179, 85-95
- **WANG JP et al.** *Pharm. Res.,* 2000, vol. 17, 782-787
- **JIA L et al.** *J. Pharm. Biomed. Anal.,* 2002, vol. 28, 65-72
- **ELIAZ RE et al.** *Cancer Res.,* 2001, vol. 61, 2592-2601